# EUROPÄISCHE PATENTSCHRIFT

(11) **EP 3 213 698 B1**
(45) Veröffentlichungstag und Bekanntmachung des Hinweises auf die Patenterteilung: **27.06.2018**
(21) Anmeldenummer: 17154211.1
(22) Anmeldetag: 01.02.2017
(51) Int. Cl.: A61B 17/17

(54) **ZIELVORRICHTUNG ZUM AUSRICHTEN EINES CHIRURGISCHEN BOHRINSTRUMENTS**
AIMING DEVICE FOR ALIGNING A SURGICAL DRILLING INSTRUMENT
DISPOSITIF CIBLE DESTINÉ À ALIGNER UN FORET CHIRURGICAL

(30) Priorität: 01.03.2016 DE 102016103642
(43) Veröffentlichungstag der Anmeldung: 06.09.2017
(73) Patentinhaber: Karl Storz SE & Co. KG, 78532 Tuttlingen (DE)
(72) Erfinder: Sauer, Michael, 78532 Tuttlingen (DE); Teufel, Felix Michael, 78532 Tuttlingen (DE); Thomas, Weishaupt, 72488 Sigmaringen (DE)

(56) Entgegenhaltungen:
- FR-A1- 2 911 264

## Beschreibung

Die vorliegende Erfindung ist auf eine Zielvorrichtung zum Ausrichten eines chirurgischen Bohrinstruments, insbesondere zum Setzen einer tibialen Bohrung zur Reparatur einer Kreuzbandruptur, gerichtet.

Zum Befestigen eines Sehnenersatzes an einem Knochen wird dieser insbesondere durchbohrt. Dabei ist die korrekte Positionierung beider Enden des Bohrkanals, insbesondere auch der Austrittsöffnung des Bohrkanals, wichtig. Dazu wurde eine Reihe von Zielvorrichtungen entwickelt, die bei ihrer vorgesehenen Verwendung eine korrekte Positionierung und Orientierung der Bohrung gewährleisten.

In DE 101 46 452 A1 ist ein Zielgerät zum Positionieren eines Bohrwerkzeugs beschrieben. Ein bogenförmiger Führungsarm des Zielgeräts kann in eine bogenförmige Führungsbahn eingesetzt und dort mittels eines Spannexzenters festgelegt werden. Ein Bohrwerkzeug kann in einer Aufnahme verschoben werden, wobei eine Sperrklinke eine Verschiebung nur in einer Richtung ermöglicht. P

In DE 10 2005 046 299 B1 ist ein Zielgerät mit einem axial verschiebbaren Zielrohr zur Führung eines Bohrwerkzeugs beschrieben. Ein Führungsschenkel ist in einem kreisbogenförmigen Schlitz geführt und mittels einer Feststellschraube fixierbar. Das axial verschiebbare Zielrohr kann mittels einer Überwurfmutter und zweier korrespondierender konischer Flächen klemmend fixiert werden.

In WO 2006/125009 A2 ist eine Vorrichtung zum Erstellen konvergenter Bohrungen beschrieben. Die Vorrichtung umfasst ein Führungsarm-Bauteil und zwei Zieleinrichtungen zum Ausrichten von Führungsstiften. Das Führungsarm-Bauteil und die Zieleinrichtungen sind entlang des Führungsbogen-Bauteils verschiebbar und mittels Schrauben fixierbar.

In DE 10 2010 024 259 A1 ist ein chirurgisches Zielgerät für die Kreuzbandrekonstruktion beschrieben. Eine Aufnahme für eine Hülse zur Führung eines Zielbohrdrahts ist entlang eines bogenförmigen Führungsarms verschiebbar und mittels eines Klemmhebels klemmend fixierbar. Die Aufnahme weist eine seitlich offene Nut auf, in die der bogenförmige Führungsarm eingesetzt ist, und aus der der bogenförmige Führungsarm seitlich entnommen werden kann.

In US 2012/0109136 A1 ist eine Führungseinrichtung zum Ausrichten eines Führungsdrahts beschrieben. Ein Handgriff weist einen gekrümmten Schlitz auf, in dem ein gekrümmter Arm bewegbar und durch eine lösbare Arretierungseinrichtung arretierbar ist. In US 8,690,885 B2 ist ein Zielgerät beschrieben. Das Zielgerät weist einen Handgriff mit einem bogenförmigen Schlitz auf. Ein bogenförmiger Abschnitt eines Arms des Zielgeräts ist in dem Schlitz geführt und mittels eines Fixierungsknopfs fixierbar. Eine weitere Zielvorrichtung ist in FR 2 911 264 A1 beschrieben. Eine Aufgabe der vorliegenden Erfindung besteht darin, eine verbesserte Zielvorrichtung zum Ausrichten eines chirurgischen Bohrinstruments zu schaffen.

Diese Aufgabe wird durch den Gegenstand des unabhängigen Anspruchs gelöst. Weiterbildungen sind in den abhängigen Ansprüchen angegeben.

Ausführungsformen der vorliegenden Erfindung beruhen auf der Idee, bei einer Zielvorrichtung, bei der eine Führungseinrichtung zum Führen eines chirurgischen Bohrinstruments und eine Zeigereinrichtung durch zwei relativ zueinander verschiebbare Bauteile miteinander verbunden sind, zur Arretierung der beiden Bauteile eines der beiden Bauteile durch eine Arretiereinrichtung elastisch zu verformen, um eine Klemmung bzw. eine reibschlüssige Verbindung zwischen den zwei Bauteilen zu erzielen. Die Arretierung erfolgt also nicht durch eine unmittelbare Klemmung bzw. reibschlüssige Verbindung zwischen einer Schraube oder einem Exzenter am einen Bauteil und einer gegenüberliegenden Fläche am anderen Bauteil. Vielmehr verformt die Arretiereinrichtung eines der Bauteile elastisch derart, dass dieses derart gegen das andere Bauteil gedrückt wird, dass eine Klemmung bzw. eine reibschlüssige Verbindung entsteht.

Eine Zielvorrichtung zum Ausrichten eines chirurgischen Bohrinstruments umfasst eine Zeigereinrichtung zur Anordnung an einem Ort an einem Knochen, an dem eine zu erstellende Bohrung austreten soll, eine Führungseinrichtung zum Führen des chirurgischen Bohrinstruments und eine einstellbare Verbindungseinrichtung mit einem ersten Ende, das mit der Führungseinrichtung verbunden ist, und einem zweiten Ende, das mit der Zeigereinrichtung verbunden ist, wobei die einstellbare Verbindungseinrichtung ein erstes Bauteil, ein zweites Bauteil und eine Arretiereinrichtung umfasst, wobei das zweite Bauteil entlang eines vorbestimmten Pfads relativ zu dem ersten Bauteil bewegbar ist, und wobei das erste Bauteil durch die Arretiereinrichtung derart elastisch verformbar ist, dass das zweite Bauteil relativ zu dem ersten Bauteil durch Klemmung arretierbar ist.

Die Zielvorrichtung ist insbesondere vorgesehen und ausgebildet, um das Setzen einer tibialen Bohrung zur Reparatur einer Kreuzbandruptur zu unterstützen. Die Zeigereinrichtung ist vorgesehen und ausgebildet, um eine visuelle und/oder taktile Prüfung der Positionierung der Zeigereinrichtung zu vereinfachen. Die Zeigereinrichtung umfasst insbesondere einen Zielhaken, eine (oder mehrere) Spitzen und/oder eine oder mehrere andere Einrichtungen, die eine Positionierung der Zeigereinrichtung vereinfachen. Ferner kann die Zeigereinrichtung so ausgebildet sein, dass sie vorübergehend kraft- und/oder formschlüssig an einer Oberfläche eines Knochens gehalten und ein Verrutschen bzw. eine unbeabsichtigte Änderung ihrer Position verhindert werden kann.

Die Führungseinrichtung umfasst insbesondere ein oder mehrere Rohre oder Hülsen zur Führung des chirurgischen Bohrinstruments. Die Führungseinrichtung weist insbesondere einen Kanal auf, dessen Querschnitt derart an den Querschnitt des chirurgischen Bohrinstruments, für das die Zielvorrichtung vorgesehen ist, angepasst ist, dass das chirurgische Bohrinstrument spiel- und reibungsarm in der Führungseinrichtung geführt ist.

Die einstellbare Verbindungseinrichtung weist insbesondere auch die Funktion eines Handgriffs auf, mittels dessen die Zielvorrichtung mit einer Hand gehalten und geführt werden kann. Insbesondere ist das erste Bauteil als Griffbauteil ausgebildet. Die Arretiereinrichtung oder zumindest ein Bedienelement bzw. mechanischer Befehlsgeber der Arretiereinrichtung ist insbesondere am ersten Bauteil angeordnet. Alternativ kann die Arretiereinrichtung oder zumindest ein Bedienelement der Arretiereinrichtung an dem zweiten Bauteil angeordnet sein.

Das erste Bauteil ist insbesondere mit der Führungseinrichtung starr oder beweglich verbunden. Insbesondere ist die Führungseinrichtung relativ zu dem ersten Bauteil entlang eines vorbestimmten Pfads verschiebbar. Der vorbestimmte Pfad, entlang dessen die Führungseinrichtung relativ zum ersten Bauteil verschiebbar ist, ist insbesondere gerade und parallel zu dem Pfad oder identisch mit dem vorbestimmten Pfad, entlang dessen ein chirurgisches Bohrinstrument durch die Führungseinrichtung geführt werden kann. Das zweite Bauteil ist insbesondere mit der Zeigereinrichtung starr verbunden. Alternativ sind das erste Bauteil mit der Zeigereinrichtung und das zweite Bauteil mit der Führungseinrichtung mechanisch verbunden.

Der vorbestimmte Pfad, entlang dessen das zweite Bauteil relativ zu dem ersten Bauteil bewegbar ist, ist insbesondere formschlüssig spiel- und reibungsarm definiert. Der vorbestimmte Pfad, entlang dessen das zweite Bauteil relativ zu dem ersten Bauteil bewegbar ist, ist insbesondere kreisbogenförmig. Der Mittelpunkt des Kreisbogens liegt insbesondere an der Zeigereinrichtung, genauer an einem Ort an der Zeigereinrichtung, der zur Anordnung an einem Ort an einer Oberfläche eines Knochens, an dem eine zu erstellende Bohrung austreten soll, vorgesehen ist. Der Mittelpunkt des Kreisbogens liegt insbesondere auf einer Symmetrieachse der Führungseinrichtung oder auf einer Symmetrieachse eines in vorgesehener Weise in die Führungseinrichtung eingeführten chirurgischen Bohrinstruments. Die Verbindungseinrichtung kann eine Variation und Einstellung der Orientierung der Zeigereinrichtung relativ zu der Führungseinrichtung ohne eine Veränderung der Position des von der Zeigereinrichtung angezeigten Orts relativ zur Führungseinrichtung ermöglichen.

Im Vergleich zu einer Schraube oder einer anderen Arretiereinrichtung, die unmittelbar auf das zweite Bauteil einwirkt und mit diesem eine reibschlüssige Verbindung herstellt, weist eine Arretierung durch elastische Verformung des ersten Bauteils eine Reihe von Vorteilen auf. Insbesondere kann eine Beschädigung einer Oberfläche des zweiten Bauteils durch die Arretiereinrichtung verhindert werden. Ferner muss das zweite Bauteil keinen Oberflächenbereich, der zur unmittelbaren Anlage der Arretiereinrichtung geeignet ist, aufweisen. Indem die Arretiereinrichtung nicht unmittelbar auf das zweite Bauteil einwirkt, sondern lediglich das erste Bauteil elastisch verformt, kann ferner eine unbeabsichtigte Bewegung des zweiten Bauteils relativ zu dem ersten Bauteil während des Arretierens verhindert werden. Umgekehrt können Kräfte, die die Arretierung belasten, oder Bewegungen des zweiten Bauteils relativ zu dem ersten Bauteil aufgrund von Spiel nicht oder nicht ohne Weiteres zum Lösen der Arretiereinrichtung führen.

Bei einer Zielvorrichtung, wie sie hier beschrieben ist, weist das erste Bauteil insbesondere eine Ausnehmung auf, in der das zweite Bauteil entlang des vorbestimmten Pfads verschiebbar geführt ist, wobei das erste Bauteil im Bereich der Ausnehmung durch die Arretiereinrichtung derart elastisch verformbar ist, dass das zweite Bauteil in der Ausnehmung durch Klemmung arretierbar ist.

Die Ausnehmung im ersten Bauteil ist insbesondere eine Nut mit einem entlang des vorbestimmten Pfads konstanten oder im Wesentlichen konstanten Querschnitt. Die Nut ist insbesondere kreisbogenförmig. Die Kontur des Querschnitts der Nut ist insbesondere C-förmig oder im Wesentlichen C-förmig mit gekrümmten und/oder geraden Abschnitten.

Durch die Arretiereinrichtung ist insbesondere der Querschnitt der Nut verformbar. Die Arretiereinrichtung ist insbesondere so angeordnet und ausgebildet, dass sie den Querschnitt der Nut oder der anderen Ausnehmung an einem Ort, an dem bei vorgesehener Verwendung immer das zweite Bauteil innerhalb der Nut oder der anderen Ausnehmung angeordnet ist, verformt.

Alternativ oder zusätzlich kann das zweite Bauteil eine Nut oder eine andere Ausnehmung, in der das erste Bauteil entlang des vorbestimmten Pfads verschiebbar geführt ist, aufweisen. In diesem Fall kann insbesondere der Querschnitt des ersten Bauteils durch die Arretiereinrichtung derart - elastisch - vergrößert werden, dass das erste Bauteil in der Ausnehmung durch Klemmung arretierbar ist. Die Arretiereinrichtung ist insbesondere so angeordnet und ausgebildet, dass sie den Querschnitt des ersten Bauteils an einem Ort, der bei vorgesehener Verwendung immer innerhalb der Nut oder der anderen Ausnehmung angeordnet ist, verformt.

Eine Ausnehmung, insbesondere eine Nut mit einem nicht kreisförmigen Querschnitt kann einerseits einfach herstellbar sein und andererseits eine zuverlässige, spiel- und reibungsarme Führung ermöglichen.

Bei einer Zielvorrichtung, wie sie hier beschrieben ist, weist das erste Bauteil insbesondere einen elastischen Abschnitt auf, der einen Teil der Oberfläche der Ausnehmung bildet.

Der elastische Abschnitt bildet insbesondere einen Teil einer inneren Oberfläche der Ausnehmung, der einem anderen Teil der inneren Oberfläche der Ausnehmung gegenüberliegt, beispielsweise parallel gegenüberliegt.

Das erste Bauteil ist insbesondere so ausgebildet, dass das zweite Bauteil auch in Abwesenheit des elastischen Abschnitts, beispielsweise vor dessen Montage oder nach dessen Demontage, formschlüssig an dem ersten Bauteil gehalten ist. Beispielsweise weisen eine Ausnehmung in dem ersten Bauteil und das zweite Bauteil - soweit es in der Ausnehmung anzuordnen ist - jeweils einen im Wesentlichen rechteckigen Querschnitt auf. In diesem Fall sind das erste Bauteil und das zweite Bauteil insbesondere so ausgebildet, dass sie einander - von Spiel abgesehen - an jeder der vier Seiten der Querschnitte berühren.

Bei einer Zielvorrichtung, wie sie hier beschrieben ist, wird der elastische Abschnitt des ersten Bauteils insbesondere durch ein separat hergestelltes und danach angefügtes Bauelement gebildet.

Das den elastischen Abschnitt des ersten Bauteils bildende separat hergestellte Bauelement ist insbesondere durch eine Schraubverbindung mit einer, zwei oder mehr Schrauben mit dem oder den übrigen Bauelementen, die das erste Bauteil bilden, mechanisch starr verbunden. Alternativ oder zusätzlich kann das den elastischen Abschnitt des ersten Bauteils bildende Bauelement auf andere Weise formschlüssig und/oder durch eine Klebung, eine Löt- oder Schweißverbindung oder auf andere Weise stoffschlüssig mit dem oder den übrigen Bauelementen, die das erste Bauteil bilden, mechanisch starr verbunden sein.

Die Ausbildung des elastischen Abschnitts aus einem separat hergestellten Bauelement ermöglicht eine Verwendung verschiedener Materialen. Insbesondere kann das den elastischen Abschnitt bildende separat hergestellte Bauelement einen Kunststoff oder ein Metall aufweisen, dessen Oberflächeneigenschaften eine reibschlüssige Arretierung begünstigen und/oder das eine höhere Elastizität aufweist.

Bei einer Zielvorrichtung, wie sie hier beschrieben ist, ist der elastische Abschnitt insbesondere zungenförmig ausgebildet und erstreckt sich entlang der Ausnehmung.

Der elastische Abschnitt weist insbesondere ein erstes Ende auf, das starr (und optional monolithisch) mit dem oder den übrigen Bauelementen des ersten Bauteils verbunden ist. Das zweite Ende des elastischen Abschnitts ist mittels der Arretiereinrichtung und aufgrund der elastischen Eigenschaften des elastischen Abschnitts relativ zum ersten Ende innerhalb eines vorbestimmten Bereichs bewegbar.

Bei einer Zielvorrichtung, wie sie hier beschrieben ist, weist der elastische Abschnitt insbesondere zwei elastisch verformbare balkenförmige Bereiche auf.

Die zwei elastisch verformbaren balkenförmigen Bereiche können jeweils abschnittsweise gerade oder gekrümmt sein. Insbesondere sind die zwei elastisch verformbaren balkenförmigen Bereich jeweils kreisbogenförmig oder im Wesentlichen kreisbogenförmig. Die zwei elastisch verformbaren balkenförmigen Bereiche sind insbesondere im Wesentlichen parallel zueinander angeordnet. Insbesondere schließen die zwei elastisch verformbaren balkenförmigen Bereiche einen Winkel ein, der nicht größer als 2 Grad oder nicht größer als 5 Grad oder nicht größer als 10 Grad ist.

Die zwei elastisch verformbaren balkenförmigen Bereiche werden insbesondere gebildet, indem zwischen diesen eine Ausnehmung gebildet wird. Die Querschnitte der zwei elastisch verformbaren balkenförmigen Bereiche sind beispielsweise jeweils schmal und länglich, beispielsweise rechteckig. Die Abmessungen der elastisch verformbaren balkenförmigen Bereiche in einer Richtung orthogonal zu dem Oberflächenbereich des elastischen Abschnitts, der zur Anlage am zweiten Bauteil vorgesehen ist, sind insbesondere kleiner oder deutlicher kleiner als die übrigen Abmessungen der elastisch verformbaren balkenförmigen Bereiche. Dies kann eine deutlich größere Elastizität des elastischen Abschnitts in der Richtung, in der er durch die Arretiereinrichtung verformt wird, als in einer dazu orthogonalen Richtung bewirken.

Bei einer Zielvorrichtung, wie sie hier beschrieben ist, weist die die Arretiereinrichtung insbesondere ein entlang eines vorbestimmten Pfads bewegbares Bedienelement auf.

Das Bedienelement bzw. der mechanische Befehlsgeber zur Steuerung der Arretiereinrichtung ist insbesondere entlang eines geraden Pfads verschiebbar. Eine Verschiebung eines Bedienelements kann vor allem bei einer einhändigen Bedienung bzw. Handhabung der Zielvorrichtung einfacher sein als ein Drehen einer Schraube oder ein Schwenken eines Hebels. Insbesondere kann eine Verschiebung des Bedienelements mittels eines Fingers bequem möglich sein.

Bei einer Zielvorrichtung, wie sie hier beschrieben ist, ist das bewegbare Bedienelement insbesondere in einer Nut in dem ersten Bauteil geführt.

Das bewegbare Bedienelement weist insbesondere einen Steg auf, der in die Nut in dem ersten Bauteil eingreift. Die Nut und der Steg sind so ausgebildet, dass der Steg in der Nut spiel- und reibungsarm geführt ist. Der Steg kann abhängig von seiner Anordnung an dem bewegbaren Bedienelement einen geraden oder einen abgewinkelten Querschnitt aufweisen.

Bei einer Zielvorrichtung, wie sie hier beschrieben ist, weist das verschiebbare Bedienelement insbesondere eine Nut auf, in der ein Steg an dem ersten Bauteil geführt ist.

Bei einer Zielvorrichtung, wie sie hier beschrieben ist, ist das Bedienelement insbesondere durch den elastischen Abschnitt des ersten Bauteils formschlüssig in der Nut in dem ersten Bauteil gehalten.

Insbesondere wenn der elastische Abschnitt als zunächst separat hergestelltes und danach angefügtes Bauelement ausgebildet ist, kann durch den elastischen Abschnitt das Bedienelement an dem ersten Bauteil gehalten werden. Nach Trennung des elastischen Abschnitts von dem oder den übrigen Bauelementen des ersten Bauteils kann das Bedienelement vom ersten Bauteil getrennt werden. Dies kann eine Reinigung der gesamten Zielvorrichtung vereinfachen.

Bei einer Zielvorrichtung, wie sie hier beschrieben ist, umfasst die Arretiereinrichtung insbesondere einen Exzenter oder einen Nocken an einem schwenkbaren Bauelement.

Ein Exzenter oder ein Nocken können ein Drehmoment an dem schwenkbaren Bauelement in eine Normalkraft zwischen einer Oberfläche des Exzenters oder des Nockens einerseits und dem elastischen Abschnitt des ersten Bauteils andererseits übersetzen.

Bei einer Zielvorrichtung, wie sie hier beschrieben ist, sind insbesondere das schwenkbare Bauelement und ein Hebel starr verbunden, wobei der Hebel und das bewegbare Bedienelement mittels eines Pleuels mechanisch gekoppelt sind.

Der Hebel ist insbesondere einstückig oder monolithisch mit dem schwenkbaren Bauelement und dem Exzenter oder Nocken ausgebildet. Der Pleuel ist insbesondere bei Herstellung der Arretierung auf Schub und beim Lösen der Arretierung auf Zug beansprucht. Der Pleuel bewirkt eine Kopplung einer linearen Bewegung des Bedienelements mit einer Schwenkbewegung des schwenkbaren Bauelements. Durch Wahl der Längen des Hebels und des Pleuels, durch Wahl des Winkels, in dem der Hebel relativ zu dem Exzenter oder Nocken an dem schwenkbaren Bauelement angeordnet ist, und durch Wahl weiterer geometrischer Eigenschaften des schwenkbaren Elements, des Pleuels und des bewegbaren Bedienelements kann ein während des Herstellens oder Lösens der Arretierung variierendes Übersetzungsverhältnis erzielt werden.

Bei einer Zielvorrichtung, wie sie hier beschrieben ist, weist das schwenkbare Bauelement insbesondere einen Zahn, der in eine Ausnehmung an dem bewegbaren Bedienelement eingreift, oder mehrere Zähne, die in eine Zahnstange an dem bewegbaren Bedienelement eingreifen, auf.

Dies ermöglicht eine mechanische Kopplung einer linearen Bewegung des bewegbaren Bedienelements und einer Schwenkbewegung des schwenkbaren Bauelements ohne das Erfordernis eines Pleuels und mit einem konstanten oder im Wesentlichen konstanten Übersetzungsverhältnis.

Bei einer Zielvorrichtung, wie sie hier beschrieben ist, weist das zweite Bauteil insbesondere eine Nut, in die das erste Bauteil eingreift, auf, wobei die Nut an dem zweiten Bauteil an dem dem ersten Bauteil zugewandten Ende so endet, dass das zweite Bauteil nicht von dem ersten Bauteil getrennt werden kann.

Insbesondere ist ein Vorsprung an dem elastischen Abschnitt am ersten Bauteil vorgesehen, der in die Nut in dem zweiten Bauteil eingreift. In diesem Fall kann ggf. nach einer Demontage des elastischen Abschnitts das zweite Bauteil von dem ersten Bauteil getrennt werden.

Bei einer Zielvorrichtung, wie sie hier beschrieben ist, ist die Führungseinrichtung insbesondere relativ zu dem ersten Ende der einstellbaren Verbindungseinrichtung entlang eines vorbestimmten Pfads bewegbar, wobei eine weitere Arretiereinrichtung zur Arretierung der Führungseinrichtung vorgesehen ist, und wobei die weitere Arretiereinrichtung so ausgebildet ist, dass eine Bewegung der Führungseinrichtung zu der Zeigereinrichtung hin innerhalb eines vorbestimmten Bereichs jederzeit möglich ist, und dass eine Bewegung der Führungseinrichtung von der Zeigereinrichtung weg nur bei Betätigung eines weiteren Bedienelements möglich ist.

Der vorbestimmte Pfad, entlang dessen die Führungseinrichtung relativ zu dem ersten Ende der Verbindungseinrichtung bewegbar ist, ist insbesondere gerade.

Eine Ausgestaltung der weiteren Arretiereinrichtung derart, dass ohne eine Betätigung eines zugeordneten weiteren Bedienelements eine Bewegung der Führungseinrichtung nur zu der Zeigereinrichtung hin möglich ist, kann eine schnelle und sichere Anbringung der Zielvorrichtung an einem Knochen ermöglichen.

Bei einer Zielvorrichtung, wie sie hier beschrieben ist, ist die weitere Arretiereinrichtung insbesondere ausgebildet, um eine Bewegung der Führungseinrichtung von der Zeigereinrichtung weg allein durch Reibschluss zu unterbinden.

Der vorbestimmte Pfad, entlang dessen die Führungseinrichtung relativ zu dem ersten Ende der Verbindungseinrichtung bewegbar ist, ist insbesondere gerade. Die Arretiereinrichtung ist insbesondere so ausgebildet, dass sie eine Bewegung der Führungseinrichtung zu der Zeigereinrichtung hin innerhalb eines vorbestimmten Bereichs jederzeit ermöglicht und eine Bewegung der Führungseinrichtung von der Zeigereinrichtung weg nur bei Betätigung eines Bedienelements ermöglicht.

Weitere Merkmale, Eigenschaften und Funktionen der Zielvorrichtungen können denjenigen der übrigen hier beschriebenen Zielvorrichtungen entsprechen.

Eine Ausbildung der Arretiereinrichtung für die Führungseinrichtung derart, dass sie eine Bewegung der Führungseinrichtung von der Zeigereinrichtung weg allein durch Reib- bzw. Kraftschluss unterbindet, jedoch eine Bewegung der Führungseinrichtung zu der Zeigereinrichtung hin innerhalb eines vorbestimmten Bereichs insbesondere jederzeit ermöglicht, kann eine einfache, schnelle und sichere Anbringung der Zielvorrichtung an einem Knochen ermöglichen. Die Unterbindung einer Bewegung der Führungseinrichtung von der Zeigereinrichtung weg allein durch Reibschluss kann eine stufenlose Positionierung der Führungseinrichtung ermöglichen. Eine stufenlose Positionierung ist beispielsweise bei einer rastenden Arretierung der Führungseinrichtung nicht möglich.

Bei einer Zielvorrichtung, wie sie hier beschrieben ist, umfasst die weitere Arretiereinrichtung insbesondere einen Hebel, der um eine weitere Schwenkachse schwenkbar ist, an dem Hebel eine Reibfläche zum Anliegen an einem von der weiteren Schwenkachse abgewandten Oberflächenbereich der Führungseinrichtung und eine elastische Einrichtung zum Bewegen der Reibfläche von der Zeigereinrichtung weg.

Dabei ist der Abstand der Zeigereinrichtung von einer ersten Ebene, die zu dem vorbestimmten Pfad der Führungseinrichtung orthogonal ist und einen vorgesehenen Berührungspunkt zwischen der Reibfläche an dem Hebel und der Führungseinrichtung enthält, insbesondere größer als der Abstand der Zeigereinrichtung von einer zweiten Ebene, die zu dem vorbestimmten Pfad der Führungseinrichtung orthogonal ist und die weitere Schwenkachse des Hebels enthält.

Bei einer Zielvorrichtung, wie sie hier beschrieben ist, umfasst die weitere Arretiereinrichtung insbesondere einen Hebel, der um eine weitere Schwenkachse schwenkbar ist, an dem Hebel eine Reibfläche zum Anliegen an einem der weiteren Schwenkachse zugewandten Oberflächenbereich der Führungseinrichtung und eine elastische Einrichtung zum Bewegen der Reibfläche von der Zeigereinrichtung weg.

Dabei ist der Abstand der Zeigereinrichtung von einer ersten Ebene, die zu dem vorbestimmten Pfad der Führungseinrichtung orthogonal ist und einen vorgesehenen Berührungspunkt zwischen der Reibfläche an dem Hebel und der Führungseinrichtung enthält, insbesondere kleiner als der Abstand der Zeigereinrichtung von einer zweiten Ebene, die zu dem vorbestimmten Pfad der Führungseinrichtung orthogonal ist und die weitere Schwenkachse des Hebels enthält.

Vereinfacht dargestellt, liegt entweder die Reibfläche an der von der Schwenkachse des Hebels abgewandten Seite der Führungseinrichtung an einem Punkt an, der weiter proximal angeordnet ist als die Schwenkachse des Hebels, oder die Reibfläche liegt an einer der Schwenkachse des Hebels zugewandten Seite der Führungseinrichtung an und berührt die Führungseinrichtung an einem Punkt, der weiter distal angeordnet ist als die Schwenkachse des Hebels. In beiden Fällen kann die Führungseinrichtung nach distal, d.h. zu der Zieleinrichtung hin verschoben werden, weil dabei nur die durch die elastische Einrichtung erzeugte Reibungskraft zwischen der Reibfläche und der Oberfläche der Führungseinrichtung überwunden werden muss. Wird jedoch eine nach proximal orientierte Kraft auf die Führungseinrichtung ausgeübt, dann wird aufgrund der Geometrie die Reibung zwischen der Reibfläche und der Oberfläche der Führungseinrichtung sowie zwischen der Führungseinrichtung und dem ersten Ende der Verbindungseinrichtung so weit vergrößert, dass eine Bewegung der Führungseinrichtung nach proximal nicht möglich ist. Erst durch manuelle Bewegung des Hebels derart, dass die Reibfläche gegen die Rückstellkraft der elastischen Einrichtung von der Oberfläche der Führungseinrichtung weg bewegt wird, kann eine Freigabe der Führungseinrichtung bewirkt und eine Bewegung der Führungseinrichtung nach proximal ermöglicht werden.

### Kurzbeschreibung der Figuren

Nachfolgend werden Ausführungsformen anhand der beigefügten Figuren näher erläutert. Es zeigen:
- Figur 1: eine schematische Darstellung einer Zielvorrichtung zum Ausrichten eines chirurgischen Bohrinstruments;
- Figur 2: eine weitere schematische Darstellung der Zielvorrichtung aus Figur 1;
- Figur 3: eine schematische Schnittdarstellung der Zielvorrichtung aus den Figuren 1 und 2;
- Figur 4: eine weitere schematische Schnittdarstellung der Zielvorrichtung aus den Figuren 1 bis 3;
- Figur 5: eine weitere schematische Schnittdarstellung der Zielvorrichtung aus den Figuren 1 bis 4;
- Figur 6: eine weitere schematische Darstellung eines Schnitts durch die Zielvorrichtung aus den Figuren 1 bis 5.

### Beschreibung der Ausführungsformen

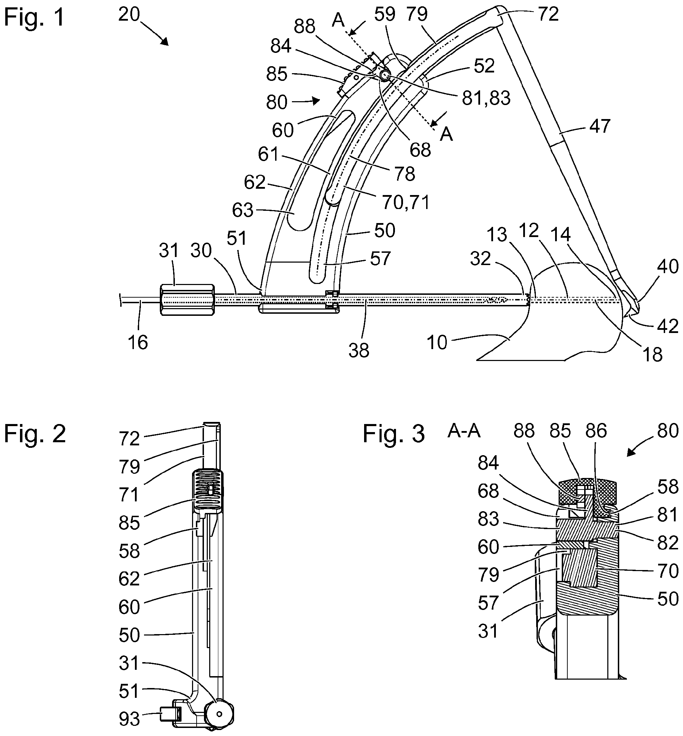

Figur 1 zeigt eine schematische Darstellung eines Knochens 10, in dem eine zylindrische Bohrung 12 zu erstellen ist. Das Bohrwerkzeug 16 ist teilweise verborgen und deshalb in gestrichelter Kontur angedeutet. Das proximale Ende des Bohrwerkzeugs 16 liegt außerhalb des dargestellten Bereichs.

Ein erstes Ende 13 der Bohrung 12, von dem ausgehend die Bohrung 12 vorangetrieben wird, kann auch bei freihändiger Führung des Bohrinstruments 16 im Idealfall präzise positioniert werden. Die Position des zweiten Endes 14 der Bohrung 12, an dem das Bohrinstrument 16 bei Vollendung der Bohrung 12 aus dem Knochen 10 wieder austreten soll, ist von der Richtung bzw. Orientierung der Achse 18 der Bohrung 12 empfindlich abhängig. Deshalb ist ohne Verwendung eines Hilfsmittels die Genauigkeit, mit der die Position des zweiten Endes 14 der Bohrung 12 im Voraus bestimmt werden kann, für viele Anwendungen unbefriedigend.

In Figur 1 ist als Hilfsmittel für die Erstellung der Bohrung 12 eine Zielvorrichtung 20 mit einer Führungseinrichtung 30 für das Bohrinstrument 16 gezeigt. Die Führungseinrichtung 30 der Zielvorrichtung 20 weist bei dem dargestellten Beispiel die Gestalt eines geraden Rohrs mit der Längs- und Symmetrieachse 38 auf. Der Querschnitt des Hohlraums der Führungseinrichtung 30 ist so an den Querschnitt des Bohrinstruments 16 angepasst, dass das Bohrinstrument 16 in der Führungseinrichtung 30 spiel- und reibungsarm geführt ist.

Optional können der Querschnitt des Hohlraums der Führungseinrichtung 30 und der Querschnitt des Bohrinstruments 16 so ausgebildet sein, dass zusätzlich eine Spülflüssigkeit durch die Führungseinrichtung 30 hindurch fließen kann.

Ein proximales Ende 31 der Führungseinrichtung 30 weist bei dem dargestellten Beispiel näherungsweise die Gestalt eines Zylinders mit regelmäßigem sechseckigem Querschnitt auf, um ein sicheres manuelles Greifen und Führen des proximalen Endes 31 der Führungseinrichtung 30 zu ermöglichen. Ein distales Ende 32 der Führungseinrichtung 30 weist bei dem dargestellten Beispiel mehrere Spitzen oder Zähne auf, die eine Positionierung des distalen Endes 32 der Führungseinrichtung 30 an der Oberfläche des Knochens 10 vereinfachen und die Wahrscheinlichkeit einer unbeabsichtigten Bewegung des distalen Endes 32 der Führungseinrichtung 30 an der Oberfläche des Knochens 10 vermindern können.

Die Zielvorrichtung 20 umfasst ferner eine Zeigereinrichtung 40 mit einer Spitze 42. Die Spitze 42 der Zeigereinrichtung 40 ist vorgesehen und ausgebildet, um auch an einer für medizinisches Personal nicht unmittelbar sichtbaren, weil abgewandten Oberfläche des Knochens 10 taktil Oberflächenstrukturen zu lokalisieren. Alternativ oder zusätzlich ist die Spitze 42 der Zeigereinrichtung 40 vorgesehen und ausgebildet, um eine Gleiten bzw. Verrutschen der Zieleinrichtung 40 an der Oberfläche eines Knochens 10 zu verhindern oder zumindest unwahrscheinlicher zu machen.

Die Zeigereinrichtung 40 ist am Ende eines Schafts 47 angeordnet, der bei dem dargestellten Beispiel gerade ist und einen kreiszylindrischen Abschnitt und einen konischen Abschnitt aufweist. Die Zeigereinrichtung 40 und der Schaft 47 können einstückig oder monolithisch hergestellt oder aus zwei oder mehr Bauelementen gefügt sein.

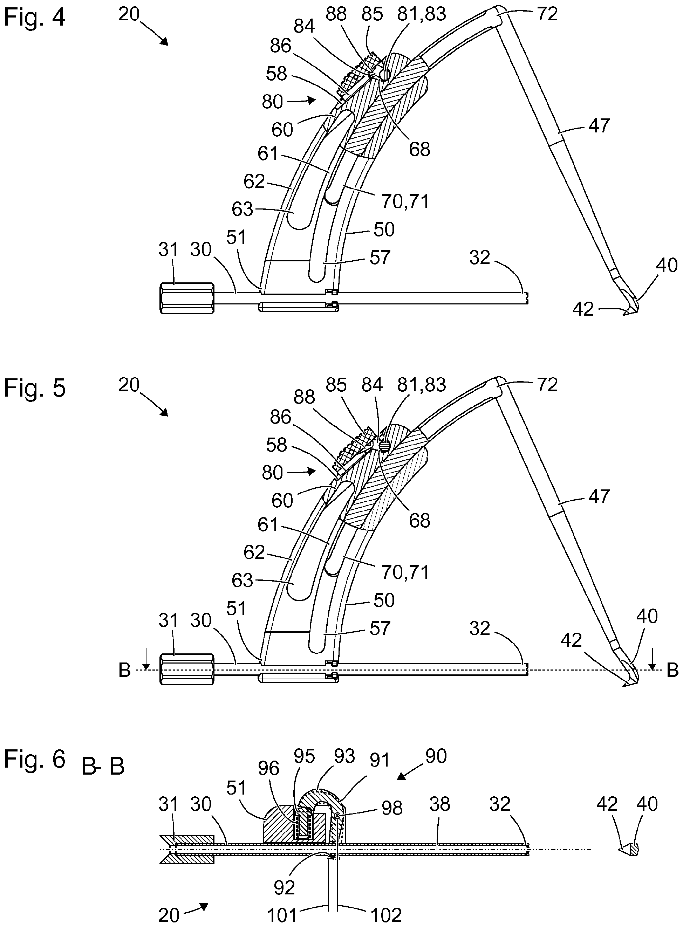

Die Zielvorrichtung 20 weist ferner eine einstellbare Verbindungseinrichtung aus einem ersten Bauteil 50 und einem zweiten Bauteil 70 auf. Ein erstes Ende 51 des ersten Bauteils 50 ist mit der Führungseinrichtung 30 mechanisch verbunden. Anhand der Figur 6 ist dargestellt, dass die Führungseinrichtung 30 relativ zu dem ersten Ende 51 des ersten Bauteils 50 in einer Richtung parallel zur Längs- und Symmetrieachse 38 der Führungseinrichtung 30 verschiebbar ist. Ein zweites Ende 52 des ersten Bauteils 50 ist dem Schaft 47 zugewandt.

Von dem zweiten Ende 52 bis nahe zu dem ersten Ende 51 des ersten Bauteils 50 erstreckt sich eine erste, kreisbogenförmige Nut 57. In der ersten Nut 57 ist das ebenfalls im Wesentlichen kreisbogenförmige zweite Bauteil 70 angeordnet. Die Krümmung und der Querschnitt der ersten Nut 57 in dem ersten Bauteil 50 und die Krümmung und der Querschnitt des kreisbogenförmigen zweiten Bauteils 70 entsprechen einander so weitgehend, dass das zweite Bauteil 70 in der ersten Nut 57 spiel- und reibungsarm geführt und entlang eines kreisbogenförmigen Pfads 78 relativ zu dem ersten Bauteil 50 bewegbar ist. Der kreisbogenförmige Pfad 78 ist durch die Gestalt der ersten Nut 57 in dem ersten Bauteil 50 und die Gestalt des zweiten Bauteils 70 vorgegeben.

Ein erstes Ende 71 des zweiten Bauteils 70 ist in der ersten Nut 57 in dem ersten Bauteil 50 angeordnet. Ein zweites Ende 72 des zweiten Bauteils 70 ist bei der in Figur 1 dargestellten Konfiguration bzw. Situation von dem ersten Bauteil 50 beabstandet. Das zweite Ende 72 des zweiten Bauteils 70 ist mit einem von der Zeigereinrichtung 40 beabstandeten Ende des Schafts 47 mechanisch starr verbunden. Beim Bewegen des zweiten Bauteils 70 entlang des kreisbogenförmigen Pfads 78 relativ zu dem ersten Bauteil 50 bleibt das erste Ende 71 des zweiten Bauteils 70 in der ersten Nut 57 in dem ersten Bauteil 50. Das zweite Ende 72 des zweiten Bauteils 70 befindet sich in einem veränderbaren Abstand von dem zweiten Ende 52 des ersten Bauteils 50.

Der Mittelpunkt des kreisbogenförmigen Pfads 78, entlang dessen das zweite Bauteil 70 relativ zu dem ersten Bauteil 50 bewegbar ist, liegt insbesondere auf oder nahe der Längs- und Symmetrieachse 38 der Führungseinrichtung 30 und nahe bei der Zeigereinrichtung 40. Dies gewährleistet, dass unabhängig von der Position des zweiten Bauteils 70 relativ zu dem ersten Bauteil 50 die Zeigereinrichtung 40 die Position des zweiten Endes 14 der zu erstellenden Bohrung 12 markiert.

Das zweite Bauteil 70 weist eine Nut 79 auf, in die ein Vorsprung 59 an dem ersten Bauteil 50 eingreift. Die Nut 79 erstreckt sich nicht ganz über die gesamte Länge des zweiten Bauteils 70. Der Eingriff des Vorsprungs 59 an dem ersten Bauteil in die Nut 79 an dem zweiten Bauteil stellt sicher, dass das erste Ende 71 des zweiten Bauteils 70 in der ersten Nut 57 in dem ersten Bauteil 50 verbleibt, und verhindert, dass das zweite Bauteil 70 ganz aus der Nut 57 in dem ersten Bauteil 50 entnommen und somit von dem ersten Bauteil 50 getrennt werden kann. Das erste Bauteil 50 und das zweite Bauteil 70 bilden somit eine zwar veränderbare bzw. einstellbare, aber nicht ohne Weiteres trennbare Einheit.

Das erste Bauteil 50 weist einen elastischen Abschnitt 60 auf. Der elastische Abschnitt 60 ist zungenförmig und erstreckt sich im Wesentlichen parallel zu der ersten Nut 57 in dem ersten Bauteil 50. Der elastische Abschnitt 60 umfasst insbesondere einen ersten elastischen Steg 61 und einen zweiten elastischen Steg 62. Die Stege 61, 62 werden insbesondere durch Erzeugung einer Ausnehmung 63 zwischen den Stegen 61, 62 gebildet.

Das dem ersten Ende 51 des ersten Bauteils 50 zugewandte Ende des elastischen Abschnitts 60 ist starr mit dem übrigen ersten Bauteil 50 verbunden. Die elastische Verformbarkeit der Stege 61, 62 ermöglicht innerhalb eines vorbestimmten Bereichs eine elastische Verformung des elastischen Abschnitts 60 und eine Bewegung des am zweiten Ende 52 des ersten Bauteils 50 angeordneten Endes des elastischen Abschnitts 60. Bei dem dargestellten Beispiel weisen der erste elastische Steg 61 und der zweite elastische Steg 62 des elastischen Abschnitts 60 jeweils im Wesentlichen die Gestalt eines kreisbogenförmig gekrümmten Balkens mit im Wesentlichen rechteckigem Querschnitt auf.

Die Abmessungen der Querschnitte der elastischen Stege 61, 62 in Richtung orthogonal zur Zeichenebene der Figur 1 sind größer oder deutlich größer als die Abmessungen der elastischen Stege 61, 62 in Richtung parallel zur Zeichenebene der Figur 1. Deshalb ist der elastische Abschnitt 60, insbesondere dessen dem zweiten Ende 52 des ersten Bauteils 50 zugewandtes Ende, in einer Richtung parallel zur Zeichenebene der Figur 1 und im Wesentlichen orthogonal zu dem Pfad 78 deutlich leichter verformbar bzw. auslenkbar als in Richtung orthogonal zur Zeichenebene der Figur 1.

Von dem elastischen Abschnitt 60 abgesehen ist das erste Bauteil 50 aufgrund seiner deutlich größeren Querschnitte und/oder aufgrund eines weniger elastischen Materials im Wesentlichen starr bzw. unelastisch.

Der elastische Abschnitt 60 und der starre Abschnitt des ersten Bauteils 50 können einstückig bzw. monolithisch und gleichzeitig hergestellt sein. Alternativ kann der elastische Abschnitt 60 zunächst als separates Bauteil hergestellt und dann - wie in Figur 1 durch eine Trennlinie nahe dem ersten Ende 51 des ersten Bauteils 50 und links der ersten Nut 57 angedeutet - mit diesem gefügt sein. Insbesondere kann der elastische Abschnitt 60 durch Schrauben und/oder auf andere formschlüssige, stoffschlüssige und/oder kraft- bzw. reibschlüssige Weise mit dem übrigen ersten Bauteil 50 gefügt sein. In diesem Fall kann der elastische Abschnitt 60 ein anderes Material als das übrige erste Bauteil 50 aufweisen, insbesondere ein Material mit höherer Elastizität. Alternativ resultiert die Elastizität des elastischen Abschnitts 60 ausschließlich aus seiner Geometrie, insbesondere den Querschnitten der elastischen Stege 61, 62.

Der elastische Abschnitt 60, insbesondere der erste elastische Steg 61 des elastischen Abschnitts 60, bildet einen Teil der inneren Oberfläche der ersten Nut 57 in dem ersten Bauteil 50. Der elastische Abschnitt 60 des ersten Bauteils 50 liegt somit an dem zweiten Bauteil 70 an oder kann an dem zweiten Bauteil 70 anliegen.

Die Zielvorrichtung 20 umfasst ferner eine erste Arretiereinrichtung 80, mittels derer der elastische Abschnitt 60 des ersten Bauteils 50 gegen das zweite Bauteil 70 gepresst werden kann, um das zweite Bauteil 70 in der ersten Nut 57 im ersten Bauteil 50 reib- bzw. kraftschlüssig zu arretieren.

Die erste Arretiereinrichtung 80 umfasst ein schwenkbares Bauelement 81, das um eine zur Zeichenebene der Figur 1 orthogonale Achse schwenkbar ist, und von dem in Figur 1 nur ein Exzenter 83 und ein Hebel 84 sichtbar sind. Die Arretiereinrichtung 80 umfasst ferner einen Schieber 85 als ein erstes Bedienelement. Der Schieber 85 ist entlang eines vorbestimmten geraden Pfads parallel zur Zeichenebene der Figur 1 relativ zu dem ersten Bauteil 50 der einstellbaren Verbindungseinrichtung der Zielvorrichtung 20 bewegbar. Ein Pleuel 88 ist weitgehend innerhalb eines zu dem ersten Bauteil 50 hin offenen Hohlraums in dem Schieber 85 angeordnet und deshalb in Figur 1 nur zu einem geringen Teil sichtbar und dargestellt. Der Pleuel 88 verbindet den Schieber 85 mit einem vom Exzenter 83 und der Schwenkachse des schwenkbaren Bauelements 81 abgewandten Ende des Hebels 84. Der Pleuel 88 koppelt eine Translationsbewegung des Schiebers 85 mit einer Rotationsbewegung des schwenkbaren Bauelements 81, insbesondere des Hebels 84 und des Exzenters 83.

Der Exzenter 83 ist in einer Kerbe 68 in dem elastischen Abschnitt 60 des ersten Bauteils 50 angeordnet. Durch Bewegen des Schiebers 85 und das damit einhergehende Schwenken des Exzenters 83 kann der elastische Abschnitt 60 des ersten Bauteils 50 in Richtung orthogonal zu dem Pfad 78 bewegt und insbesondere gegen das zweite Bauteil 70 gepresst werden. Bei der in Figur 1 gezeigten Situation bzw. Konfiguration sind insbesondere der Schieber 85 in einer Position nahe dem zweiten Ende 52 des ersten Bauteils 50 angeordnet, der elastische Abschnitt 60 des ersten Bauteils 50 gegen das zweite Bauteil 70 gepresst und dadurch das zweite Bauteil in der ersten Nut 57 in dem ersten Bauteil 50 durch Klemmung, das heißt kraft- bzw. reibschlüssig arretiert.

Figur 2 zeigt eine weitere schematische Darstellung der Zielvorrichtung 20 aus Figur 1. Die Zeichenebene der Figur 2 ist orthogonal zur Zeichenebene der Figur 1 und orthogonal oder im Wesentlichen orthogonal zu der Längs- und Symmetrieachse 38 der Führungseinrichtung, von der in Figur 2 nur das proximale Ende 31 sichtbar ist. Bei dem dargestellten Beispiel ist der elastische Abschnitt 60 des ersten Bauteils 50 durch ein ursprünglich separat hergestelltes und dann mit dem übrigen ersten Bauteil 50 gefügtes Bauelement gebildet. In Figur 2 ist ferner ein Ende einer zweiten Nut 58 in dem ersten Bauteil 50 sichtbar. Ein in Figur 2 nicht sichtbarer Steg an dem Schieber 85 greift in die zweite Nut 58 ein, so dass der Schieber 85 formschlüssig an dem ersten Bauteil 50 gehalten und entlang eines vorbestimmten Pfads relativ zu dem Bauteil 50 bewegbar ist.

Am ersten Ende 51 des ersten Bauteils 50 ist ein zweites Bedienelement 93 einer zweiten Arretiereinrichtung, die in Figur 2 im Übrigen nicht sichtbar ist, angeordnet.

Figur 3 zeigt eine schematische Darstellung eines Schnitts entlang der in Figur 1 angedeuteten Ebene A-A orthogonal zu dem Pfad 78. Die Schnittebene A-A der Figur 3 enthält die Schwenkachse des bereits erwähnten schwenkbaren Bauelements 81. Die Darstellung in Figur 3 ist gegenüber den Darstellungen in den Figuren 1 und 2 vergrößert, damit Details besser sichtbar sind.

Die Schnittebene A-A der Figur 3 schneidet den elastischen Abschnitt 60 des ersten Bauteils 50 im Bereich der Kerbe 68 (vgl. Figur 1). Der in Figur 3 sichtbare Querschnitt des elastischen Abschnitts 60 ist deshalb klein. Der Querschnitt des übrigen ersten Bauteils 50 ist im Wesentlichen L-förmig und vergleichsweise massiv, um eine starre und unelastische Führung des zweiten Bauteils 70 in dem ersten Bauteil 50 zu ermöglichen.

Der Querschnitt der Nut 57 in dem ersten Bauteil 50 und der Querschnitt des zweiten Bauteils 70 sind jeweils im Wesentlichen rechteckig. Der Querschnitt des starren Abschnitts des ersten Bauteils 50 (das heißt ohne den elastischen Abschnitt 60) umgreift das zweite Bauteil 70 so, dass bezogen auf zwei orthogonale Richtungen (in Figur 3: horizontal und vertikal) jeweils das erste Bauteil 50 an zwei einander gegenüberliegenden parallelen Oberflächenabschnitten des zweiten Bauteils 70 anliegt. Das zweite Bauteil 70 ist deshalb auch ohne Berücksichtigung des elastischen Abschnitts 60 vollständig in dem ersten Bauteil 50 geführt.

Das bereits im Kontext von Figur 1 erwähnte schwenkbare Bauelement 81 umfasst eine Welle oder einen Achszapfen 82, der in einer Bohrung im starren Abschnitt des ersten Bauteils 50 spielarm gelagert ist. Ferner umfasst das schwenkbare Element 81 den bereits erwähnten Exzenter 83, der bei der in den Figuren 1 bis 3 gezeigten Situation bzw. Konfiguration den elastischen Abschnitt 60 gegen das zweite Bauteil 70 presst und damit das zweite Bauteil 70 reib- bzw. kraftschlüssig in der ersten Nut 57 im ersten Bauteil 50 arretiert. Der Pleuel 88 ist nur an seinem dem Hebel 84 am schwenkbaren Bauelement 81 zugewandten Ende durch die Schnittebene A-A der Figur 3 angeschnitten.

Der bereits erwähnte, in den Figuren 1 und 2 jedoch nicht sichtbare Steg 86 an dem Schieber 85 weist einen L-förmigen Querschnitt auf und greift in die zweite Nut 58 im ersten Bauteil 50, deren Ende in Figur 2 sichtbar ist, ein.

Figur 4 zeigt eine weitere schematische Darstellung der Zielvorrichtung 20 aus den Figuren 1 bis 3. Die Zeichenebene der Figur 4 entspricht der Zeichenebene der Figur 1. Im Unterschied zu Figur 1 ist die Zielvorrichtung 20 jedoch im Bereich der ersten Arretiereinrichtung 80 angeschnitten dargestellt. Die in Figur 4 gezeigte Situation bzw. Konfiguration entspricht der in den Figuren 1 bis 3 gezeigten.

In Figur 4 sind ein Ende der zweiten Nut 58 in dem ersten Bauteil 50 und der Steg 86 an dem Schieber 85 sichtbar.

Figur 5 zeigt eine weitere schematische Darstellung der Zielvorrichtung 20 aus den Figuren 1 bis 4. Die Art der Darstellung entspricht derjenigen der Figur 4. Im Unterschied zu Figur 4 ist die Zielvorrichtung 20 jedoch in einer anderen Situation bzw. Konfiguration dargestellt. Der Schieber 85 ist ausgehend von der in den Figuren 1 bis 4 gezeigten Position nach proximal bzw. zum ersten Ende 51 des ersten Bauteils 50 hin verschoben. Aufgrund der Kopplung des Schiebers 85 mit dem Hebel 84 und dem Exzenter 83 durch den Pleuel 88 ist der Exzenter 83 gegenüber der in den Figuren 1 bis 4 gezeigten Situation gegen den Uhrzeigersinn gedreht. Der Exzenter 83 übt deshalb eine geringere oder keine Kraft auf den elastischen Abschnitt 60 des ersten Bauteils 50 aus, so dass das zweite Bauteil 70 nicht kraft- bzw. reibschlüssig arretiert ist, sondern relativ zu dem ersten Bauteil 50 entlang des Pfads 78 (vgl. Figur 1) bewegt werden kann.

Figur 6 zeigt eine schematische Darstellung eines weiteren Schnitts durch die Zielvorrichtung 20 aus den Figuren 1 bis 5 entlang der in Figur 5 angedeuteten Ebene B-B. Die Schnittebene B-B ist orthogonal zu den Zeichenebenen der Figuren 1, 2, 4 und 5 und enthält die Längs- und Symmetrieachse 38 der Führungseinrichtung 30.

Die Schnittebene B-B schneidet die bereits im Kontext von Figur 2 erwähnte, jedoch in Figur 2 nicht sichtbare zweite Arretiereinrichtung 90. Die zweite Arretiereinrichtung 90 umfasst einen im Wesentlichen L-förmigen Hebel 91, der um eine Schwenkachse 98 orthogonal zur Schnittebene B-B der Figur 6 schwenkbar ist. Ein Ende des Hebels 91 umgreift die Führungseinrichtung 30 gabelförmig und liegt mit Reibflächen 92 an der von der Schwenkachse 98 abgewandten Seite der Führungseinrichtung 30 an. Der zweite Schenkel des Hebels 91 bildet das auch in Figur 2 sichtbare Bedienelement 93 und liegt an einem Stempel 95 an. Eine Schraubenfeder 96 drückt den Stempel 95 und damit den Hebel 91 in die in Figur 6 gezeigten Positionen. Durch manuellen Druck auf den als zweites Bedienelement 93 vorgesehenen Bereich des Hebels 91 kann der Hebel 91 gegen die elastische Rückstellkraft der Schraubenfeder 96 in eine Position geschwenkt werden, deren Konturen in Figur 6 durch gestrichelte Linien angedeutet sind.

Bei der in Figur 6 gezeigten Situation liegt ein Berührungspunkt zwischen der Reibfläche 92 am Hebel 91 einerseits und der Führungseinrichtung 30 andererseits weiter proximal (in Figur 6: weiter links) als die Schwenkachse 98. Genauer: eine erste Ebene 101, die orthogonal zur Längs- und Symmetrieachse 38 der Führungseinrichtung 30 ist und den Berührungspunkt zwischen der Reibfläche 92 am Hebel 91 und der Führungseinrichtung 30 enthält, ist vom distalen Ende 32 der Führungseinrichtung 30 weiter entfernt als eine zweite Ebene 102, die zu der ersten Ebene 101 parallel ist und die Schwenkachse 98 der zweiten Arretiereinrichtung 90 enthält.

In Folge der dargestellten Geometrie bewirkt eine nach proximal orientierte Kraft auf die Führungseinrichtung 30 eine Klemmung der Führungseinrichtung 30. Die Klemmung unterbindet reib- bzw. kraftschlüssig eine Bewegung der Führungseinrichtung 30 relativ zu dem ersten Ende 51 des ersten Bauteils der einstellbaren Verbindungseinrichtung. Nur wenn gleichzeitig Druck auf den als zweites Bedienelement 93 vorgesehenen Bereich des Hebels 91 ausgeübt und dieser in Richtung zu der in Figur 6 in gestrichelten Linien angedeuteten Löseposition geschwenkt wird, kann die Führungseinrichtung 30 nach proximal verschoben werden.

Eine Bewegung der Führungseinrichtung 30 nach distal relativ zu dem ersten Ende 51 des ersten Bauteils der einstellbaren Verbindungseinrichtung ist hingegen jederzeit möglich.

Zum Austauschen der Führungseinrichtung 30 oder zum Zerlegen der Zielvorrichtung 20 kann die Führungseinrichtung 30 nach proximal aus dem ersten Ende 51 des ersten Bauteils 50 herausgezogen werden. Dazu muss lediglich gleichzeitig Druck auf den als zweites Bedienelement 93 vorgesehenen Bereich des Hebels 91 ausgeübt und dieser in Richtung zu der in Figur 6 in gestrichelten Linien angedeuteten Löseposition geschwenkt werden.

Um die Zielvorrichtung 20 weiter zu zerlegen, kann in einem nächsten Schritt die erste Arretiereinrichtung 80 von dem ersten Bauteil 50 getrennt werden. Bei dem hier dargestellten Beispiel sind das schwenkbare Bauelement 81 und die Kerbe 68 im elastischen Abschnitt 60 des ersten Bauteils 50 so ausgebildet, dass das schwenkbare Bauelement 81 zusammen mit dem Pleuel 88 und dem Schieber 85 durch eine Bewegung in Richtung orthogonal zu den Zeichenebenen der Figuren 1, 4 und 5 und parallel zu den Zeichenebenen der Figuren 2 und 3 relativ zu dem ersten Bauteil 50 von diesem getrennt werden kann. Dabei werden der Achszapfen 82 des schwenkbaren Bauelements 81 aus der korrespondierenden Bohrung im starren Abschnitt des ersten Bauteils 50 und gleichzeitig der L-förmige Steg 86 am Schieber 85 aus der zweiten Nut 58 am ersten Bauteil 50 entnommen. Die Richtung der Bewegung ist bezogen auf die Figur 2 nach rechts und bezogen auf die Figur 3 nach links.

Der Hebel an dem schwenkbaren Bauelement 81 und die Kerbe 68 im elastischen Abschnitt 60 des ersten Bauteils 50 sind insbesondere so ausgebildet, dass die beschriebene Bewegung des schwenkbaren Bauelements 81 relativ zu dem ersten Bauteil 50 nur dann möglich ist, wenn das schwenkbare Bauelement 81 eine vorbestimmte Winkelposition einnimmt, beispielsweise die in den Figuren 1 und 4 dargestellte. Diese Winkelposition kann durch Markierungen an dem ersten Bauteil 50 und an dem Schieber 85 gekennzeichnet sein. Der Hebel an dem schwenkbaren Bauelement 81 und die Kerbe 68 im elastischen Abschnitt 60 des ersten Bauteils 50 sind insbesondere so ausgebildet, dass die beschriebene Bewegung des schwenkbaren Bauelements 81 nur in einer einzigen Winkelposition oder einem kleinen oder sehr kleinen Bereich von Winkelpositionen möglich ist. In allen anderen Winkelpositionen, insbesondere in den beiden extremen bzw. Endpositionen, ist der Hebel 84 an dem schwenkbaren Bauelement 81 durch den elastischen Abschnitt 60 des ersten Bauteils 50 an der beschriebenen Bewegung formschlüssig gehindert.

Alternativ kann eine andere Trennbarkeit der ersten Arretiereinrichtung 80 von dem ersten Bauteil 50 vorgesehen sein.

Um die Zielvorrichtung 20 noch weiter zu zerlegen, kann das zweite Bauteil 70 von dem ersten Bauteil 50 getrennt werden. Dazu wird das zweite Bauteil 70 - bezogen auf die Darstellungen in den Figuren 1, 4 und 5 - in einer Bewegung im Uhrzeigersinn aus der ersten Nut 57 in dem ersten Bauteil 50 heraus gezogen. In den in den Figuren 1 bis 5 dargestellten Situationen bzw. Konfigurationen verhindern der Eingriff des Vorsprungs 59 an dem ersten Bauteil 50 in die Nut 79 in dem zweiten Bauteil 70 und das Ende der Nut 79 in dem zweiten Bauteil 70 vor dem ersten Ende 71 des zweiten Bauteils 70 dieses Entnehmen des zweiten Bauteils 70 aus der ersten Nut 59 in dem ersten Bauteil 50. Dies gilt nicht mehr, wenn die erste Arretiereinrichtung 80 von dem ersten Bauteil 50 getrennt bzw. entfernt ist. In diesem Fall hindert der Exzenter 83 des schwenkbaren Bauelements 81 den elastischen Abschnitt 60 des ersten Bauteils 50 nicht mehr an einer Verformung nach radial außen bzw. - bezogen auf die Darstellungen in den Figuren 1, 4 und 5 - nach links oben. Durch Verformung des elastischen Abschnitts 60 des ersten Bauteils 50 kann also der Vorsprung 59 an dem elastischen Abschnitt 60 des ersten Bauteils 50 aus der Nut 79 im zweiten Bauteil 70 heraus klettern.

Ein Zusammensetzen der Zielvorrichtung 20 ist in der umgekehrten Reihenfolge und mit umgekehrten Bewegungsrichtungen möglich.

### Bezugszeichen

- 10: Knochen
- 12: zu erstellende Bohrung im Knochen
- 13: erstes Ende der Bohrung 12
- 14: zweites Ende der Bohrung 12
- 16: Bohrinstrument
- 18: Achse der zu erstellenden Bohrung 12 und vorgesehener Pfad, entlang dessen die Führungseinrichtung 30 verschiebbar ist
- 20: Zielvorrichtung
- 30: Führungseinrichtung an der Zielvorrichtung 20
- 31: proximales Ende der Führungseinrichtung 30
- 32: distales Ende der Führungseinrichtung 30
- 38: Längs- und Symmetrieachse der Führungseinrichtung 30 und vorgesehener Pfad, entlang dessen die Führungseinrichtung 30 verschiebbar ist
- 40: Zeigereinrichtung an der Zielvorrichtung 20
- 42: Spitze am distalen Ende der Zeigereinrichtung 40
- 47: Schaft, im Wesentlichen radial angeordnet
- 50: erstes Bauteil einer einstellbare Verbindungseinrichtung der Zielvorrichtung 20
- 51: erste Ende des ersten Bauteils 50
- 52: zweites Ende des ersten Bauteils 50
- 57: erste Nut im ersten Bauteil 50
- 58: zweite Nut im ersten Bauteil 50
- 59: Vorsprung am ersten Bauteil 50, in Nut 79 am zweiten Bauteil 70 eingreifend
- 60: elastischer Abschnitt des ersten Bauteils 50
- 61: erster elastischer Steg des elastischen Abschnitts 60
- 62: zweiter elastischer Steg des elastischen Abschnitts 60
- 63: Ausnehmung zwischen den Stegen 61, 62 des elastischen Abschnitts 60
- 68: Kerbe in dem elastischen Abschnitt 60
- 70: im Wesentlichen kreisbogenförmiges zweites Bauteil der Verbindungseinrichtung
- 71: erstes Ende des zweiten Bauteils 70
- 72: zweites Ende des zweiten Bauteils 70
- 78: kreisbogenförmiger Pfad, entlang dessen das zweite Bauteils 70 relativ zum ersten Bauteil 50 verschiebbar ist, durch erste Nut 57 im ersten Bauteil 50 formschlüssig definiert
- 79: Nut im zweiten Bauteil 70, den Vorsprung 59 am ersten Bauteil 50 aufnehmend
- 80: erste Arretiereinrichtung der Verbindungseinrichtung zum Arretieren des zweiten Bauteils 70 im ersten Bauteil 50
- 81: schwenkbares Bauelement der ersten Arretiereinrichtung 80
- 82: Welle oder Achszapfen am schwenkbaren Bauelement 81
- 83: Exzenter am schwenkbaren Bauelement 81
- 84: Hebel am schwenkbaren Bauelement 81
- 85: Schieber als erstes Bedienelement für erste Arretiereinrichtung 80
- 86: Steg am Schieber 81, in zweite Nut 58 am ersten Bauteil 50 eingreifend
- 88: Pleuel zwischen Schieber 85 und Hebel 84 am schwenkbaren Bauelement 81
- 90: zweite Arretiereinrichtung der Verbindungseinrichtung zum Arretieren der Führungseinrichtung 90 am ersten Bauteil 50
- 91: Hebel der zweiten Arretiereinrichtung 90
- 92: Reibfläche am Hebel 91
- 93: als zweites Bedienelement vorgesehener Bereich des Hebels 91
- 95: Stempel
- 96: Schraubenfeder
- 98: Schwenkachse der zweiten Arretiereinrichtung 90
- 101: erste Ebene
- 102: zweite Ebene

## Patentansprüche

1. **Zielvorrichtung** (20) zum Ausrichten eines chirurgischen Bohrinstruments (16), mit:
einer **Zeigereinrichtung** (40) zur Anordnung an einem Ort (14) an einem Knochen (10), an dem eine zu erstellende Bohrung (12) austreten soll;
einer **Führungseinrichtung** (30) zum Führen des chirurgischen Bohrinstruments (16);
einer einstellbaren **Verbindungseinrichtung** (50, 70, 80) mit einem ersten Ende (51), das mit der Führungseinrichtung (30) verbunden ist, und einem zweiten Ende (52), das mit der Zeigereinrichtung (40) verbunden ist,
wobei die einstellbare Verbindungseinrichtung ein **erstes Bauteil** (50), ein **zweites Bauteil** (70) und eine **Arretiereinrichtung** (80) umfasst,
wobei das zweite Bauteil (70) entlang eines vorbestimmten Pfads (78) relativ zu dem ersten Bauteil (50) bewegbar ist, **dadurch gekennzeichnet, dass** das **erste Bauteil** (50) durch die Arretiereinrichtung (80) derart **elastisch verformbar** ist, dass das zweite Bauteil (70) relativ zu dem ersten Bauteil (50) durch Klemmung arretierbar ist.

2. Zielvorrichtung (20) nach dem vorangehenden Anspruch, bei der
das erste Bauteil (50) eine **Ausnehmung** (57) aufweist, in der das zweite Bauteil (70) entlang des vorbestimmten Pfads (78) verschiebbar geführt ist,
das **erste Bauteil** (50) im Bereich der Ausnehmung (57) **durch die Arretiereinrichtung** (80) derart **elastisch verformbar** ist, dass das zweite Bauteil (70) in der Ausnehmung (57) durch Klemmung arretierbar ist

3. Zielvorrichtung (20) nach dem vorangehenden Anspruch, bei der das erste Bauteil (50) einen **elastischen Abschnitt** (60) aufweist, der einen Teil der [inneren] Oberfläche der Ausnehmung (57) bildet.

4. Zielvorrichtung nach dem vorangehenden Anspruch, bei der der elastische Abschnitt des ersten Bauteils (50) durch ein **separat hergestelltes** und danach **angefügtes Bauelement** (60) gebildet wird.

5. Zielvorrichtung (20) nach dem vorangehenden Anspruch, bei der der elastische Abschnitt (60) **zungenförmig** ausgebildet ist und sich entlang der Ausnehmung (57) erstreckt.

6. Zielvorrichtung (20) nach einem der Ansprüche 2 bis 5, bei der der elastische Abschnitt (60) zwei elastisch verformbare **balkenförmige Bereiche** (61, 62) aufweist.

7. Zielvorrichtung (20) nach einem der vorangehenden Ansprüche, bei der die Arretiereinrichtung (80) ein entlang eines vorbestimmten Pfads **bewegbares Bedienelement** (85) aufweist.

8. Zielvorrichtung (20) nach dem vorangehenden Anspruch, bei der das bewegbare **Bedienelement** (85) in einer **Nut** (58) in dem ersten Bauteil (50) geführt ist.

9. Zielvorrichtung (20) nach einem der vorangehenden Ansprüche, bei der die Arretiereinrichtung (80) einen **Exzenter** (83) oder einen **Nocken** an einem schwenkbaren Bauelement (81) umfasst.

10. Zielvorrichtung (20) nach dem vorangehenden Anspruch in Rückbezug auf Anspruch 6 oder 7, bei der
das schwenkbare Bauelement (81) und ein Hebel (84) starr verbunden sind,
der Hebel (84) und das bewegbare Bedienelement (85) mittels eines **Pleuels** (88) mechanisch gekoppelt sind.

11. Zielvorrichtung (20) nach einem der vorangehenden Ansprüche, bei der
das zweite Bauteil (70) eine **Nut** (79), in die das erste Bauteil (50) eingreift, aufweist,
die Nut (79) an dem zweiten Bauteil (70) an dem dem ersten Bauteil (50) zugewandten Ende so endet, dass das zweite Bauteil (70) nicht von dem ersten Bauteil (50) getrennt werden kann.

12. Zielvorrichtung (20) nach einem der vorangehenden Ansprüche, bei der
die **Führungseinrichtung** (30) relativ zu dem ersten Ende (51) der einstellbaren Verbindungseinrichtung (50, 60, 70, 80) entlang eines vorbestimmten Pfads (38) **bewegbar** ist,
eine weitere **Arretiereinrichtung** (90) zur Arretierung der Führungseinrichtung (30) vorgesehen ist,
die weitere Arretiereinrichtung (90) so ausgebildet ist, dass eine Bewegung der Führungseinrichtung (30) zu der Zeigereinrichtung (40) hin innerhalb eines vorbestimmten Bereichs jederzeit möglich ist, und dass eine Bewegung der Führungseinrichtung (30) von der Zeigereinrichtung (40) weg nur bei Betätigung eines weiteren Bedienelements (93) möglich ist.

13. Zielvorrichtung (20) nach dem vorangehenden Anspruch, bei der die weitere Arretiereinrichtung (90) ausgebildet ist, um eine Bewegung der Führungseinrichtung (30) von der Zeigereinrichtung (40) weg allein durch Reibschluss zu unterbinden.

14. Zielvorrichtung (20) nach dem vorangehenden Anspruch, bei der
die weitere Arretiereinrichtung (90) einen **Hebel** (91), der um eine weitere Schwenkachse (98) schwenkbar ist, an dem Hebel (91) eine Reibfläche (92) zum Anliegen an einem von der weiteren Schwenkachse (98) abgewandten Oberflächenbereich der Führungseinrichtung (30) und eine elastische Einrichtung (96) zum Bewegen der Reibfläche (92) von der Zeigereinrichtung (40) weg umfasst,
der Abstand der Zeigereinrichtung (40) von einer ersten Ebene (101), die zu dem vorbestimmten Pfad (38) der Führungseinrichtung (30) orthogonal ist und einen vorgesehenen Berührungspunkt zwischen der Reibfläche (92) an dem Hebel (91) und der Führungseinrichtung (30) enthält, größer ist als der Abstand der Zeigereinrichtung (40) von einer zweiten Ebene (102), die zu dem vorbestimmten Pfad (38) der Führungseinrichtung (30) orthogonal ist und die weitere Schwenkachse (98) des Hebels (91) enthält.

## Claims

1. Aiming device (20) for aligning a surgical drilling instrument (16), with:
a pointer mechanism (40) for arranging at a location (14) on a bone (10) where a drilled hole (12) that is to be formed is intended to emerge;
a guide mechanism (30) for guiding the surgical drilling instrument (16);
an adjustable connection mechanism (50, 70, 80) with a first end (51), which is connected to the guide mechanism (30), and a second end (52), which is connected to the pointer mechanism (40),
wherein the adjustable connection mechanism comprises a first component (50), a second component (70) and a locking mechanism (80),
wherein the second component (70) is movable relative to the first component (50) along a predetermined path (78),
**characterized in that**
the first component (50) is elastically deformable by the locking mechanism (80) in such a way that the second component (70) is lockable relative to the first component (50) by clamping.

2. Aiming device (20) according to the preceding claim, in which
the first component (50) has a recess (57) in which the second component (70) is guided movably along the predetermined path (78),
the first component (50) is elastically deformable in the region of the recess (57) by the locking mechanism (80), in such a way that the second component (70) is lockable in the recess (57) by clamping.

3. Aiming device (20) according to the preceding claim, in which the first component (50) has an elastic portion (60) that forms a part of the [inner] surface of the recess (57).

4. Aiming device according to the preceding claim, in which the elastic portion of the first component (50) is formed by a structural element (60) that is produced separately and thereafter attached.

5. Aiming device (20) according to the preceding claim, in which the elastic portion (60) is tongue-shaped and extends along the recess (57).

6. Aiming device (20) according to one of Claims 2 to 5, in which the elastic portion (60) has two elastically deformable beam-shaped regions (61, 62) .

7. Aiming device (20) according to one of the preceding claims, in which the locking mechanism (80) has an operating element (85) movable along a predetermined path.

8. Aiming device (20) according to the preceding claim, in which the movable operating element (85) is guided in a groove (58) in the first component (50) .

9. Aiming device (20) according to one of the preceding claims, in which the locking mechanism (80) comprises an eccentric (83) or a cam on a pivotable structural element (81).

10. Aiming device (20) according to the preceding claim referred back to Claim 6 or 7, in which
the pivotable structural element (81) and a lever (84) are rigidly connected,
the lever (84) and the movable operating element (85) are mechanically coupled by means of a connecting rod (88).

11. Aiming device (20) according to one of the preceding claims, in which
the second component (70) has a groove (79) in which the first component (50) engages,
the groove (79) on the second component (70) ends at the end directed towards the first component (50) such that the second component (70) cannot be separated from the first component (50).

12. Aiming device (20) according to one of the preceding claims, in which
the guide mechanism (30) is movable relative to the first end (51) of the adjustable connection mechanism (50, 60, 70, 80) along a predetermined path (38),
a further locking mechanism (90) is provided for locking the guide mechanism (30),
the further locking mechanism (90) is configured such that a movement of the guide mechanism (30) towards the pointer mechanism (40) is possible at any time within a predetermined range, and a movement of the guide mechanism (30) away from the pointer mechanism (40) is possible only upon actuation of a further operating element (93).

13. Aiming device (20) according to the preceding claim, in which the further locking mechanism (90) is configured to suppress a movement of the guide mechanism (30) away from the pointer mechanism (40) solely by frictional engagement.

14. Aiming device (20) according to the preceding claim, in which
the further locking mechanism (90) comprises a lever (91) which is pivotable about a further pivot axis (98), a friction surface (92) on the lever (91) for bearing on a surface region of the guide mechanism (30) directed away from the further pivot axis (98), and an elastic mechanism (96) for moving the friction surface (92) away from the pointer mechanism (40),
the distance of the pointer mechanism (40) from a first plane (101), which is orthogonal to the predetermined path (38) of the guide mechanism (30) and contains an intended contact point between the friction surface (92) on the lever (91) and the guide mechanism (30), is greater than the distance of the pointer mechanism (40) from a second plane (102), which is orthogonal to the predetermined path (38) of the guide mechanism (30) and contains the further pivot axis (98) of the lever (91).

## Revendications

1. Ensemble de visée (20) destiné à aligner un instrument chirurgical de forage (16) et présentant :
un dispositif de pointage (40) destiné à être placé en un emplacement (14) d'un os (10) dans lequel il faut former un alésage (12),
un dispositif de guidage (30) qui guide l'instrument chirurgical de forage (16),
un dispositif ajustable de liaison (50, 70, 80) présentant une première extrémité (51) reliée au dispositif de guidage (30) et une deuxième extrémité (52) reliée au dispositif de pointage (40),
le dispositif ajustable de liaison comportant un premier composant (50), un deuxième composant (70) et un dispositif de blocage (80),
le deuxième composant (70) pouvant être déplacé par rapport au premier composant (50) le long d'un parcours prédéterminé (78),
**caractérisé en ce que**
le premier composant (50) peut être déformé élastiquement par le dispositif de blocage (80) et **en ce que** le deuxième composant (70) peut être bloqué par rapport au premier composant (50) par serrage.

2. Ensemble de visée (20) selon la revendication précédente, dans lequel le premier composant (50) présente une découpe (57) dans laquelle le deuxième composant (70) est guidé à coulissement le long du parcours prédéterminé (78),
le premier composant (50) pouvant être déformé élastiquement au niveau de la découpe (57) par le dispositif de blocage (80) de telle sorte que le deuxième composant (70) puisse être bloqué dans la découpe (57) par serrage.

3. Ensemble de visée (20) selon la revendication précédente, dans lequel le premier composant (50) présente une partie élastique (60) qui fait partie de la surface [intérieure] de la découpe (57).

4. Ensemble de visée (20) selon la revendication précédente, dans lequel la partie élastique du premier composant (50) est formée par un composant (60) fabriqué séparément et ensuite relié.

5. Ensemble de visée (20) selon la revendication précédente, dans lequel la partie élastique (60) est configurée en languette qui s'étend le long de la découpe (57).

6. Ensemble de visée (20) selon l'une des revendications 2 à 5, dans lequel la partie élastique (60) présente deux parties élastiquement déformables (61, 62) en forme de barre.

7. Ensemble de visée (20) selon l'une des revendications précédentes, dans lequel le dispositif de blocage (80) présente un élément d'actionnement (85) qui peut être déplacé le long d'un parcours prédéterminé.

8. Ensemble de visée (20) selon la revendication précédente, dans lequel l'élément d'actionnement (85) est guidé dans une rainure (58) du premier composant (50) .

9. Ensemble de visée (20) selon l'une des revendications précédentes, dans lequel le dispositif de blocage (80) comporte un excentrique (83) ou une came disposée sur un composant pivotant (81).

10. Ensemble de visée (20) selon la revendication précédente dans la mesure où elle est subordonnée aux revendications 6 ou 7, dans lequel le composant pivotant (81) et un levier (84) sont reliés rigidement et le levier (84) ainsi que l'élément mobile d'actionnement (85) sont reliés mécaniquement l'un à l'autre au moyen d'une bielle (88).

11. Ensemble de visée (20) selon l'une des revendications précédentes, dans lequel le deuxième composant (70) présente une rainure (79) dans laquelle s'engage le premier composant (50), la rainure (79) du deuxième composant (70) se terminant sur l'extrémité tournée vers le premier composant (50) de telle sorte que le deuxième composant (70) ne puisse être séparé du premier composant (50).

12. Ensemble de visée (20) selon l'une des revendications précédentes, dans lequel le dispositif de guidage (30) peut être déplacé par rapport à la première extrémité (51) du dispositif ajustable de liaison (50, 60, 70, 80) le long d'un parcours prédéterminé (38), dans lequel un autre dispositif de blocage (90) est prévu pour bloquer le dispositif de guidage (30) et dans lequel l'autre dispositif de blocage (90) est configuré de telle sorte qu'il permet toujours un déplacement du dispositif de guidage (30) en direction du dispositif de pointage (40) à l'intérieur d'une partie prédéterminée et qu'un éloignement du dispositif de guidage (30) par rapport au dispositif de pointage (40) ne soit possible que si un autre élément d'actionnement (93) est actionné.

13. Ensemble de visée (20) selon la revendication précédente, dans lequel le l'autre dispositif de blocage (90) est configuré pour empêcher un éloignement du dispositif de guidage (30) par rapport au dispositif de pointage (40) uniquement par correspondance de frottement.

14. Ensemble de visée (20) selon la revendication précédente, dans lequel l'autre dispositif de blocage (90) comporte un levier (91) apte à pivoter autour d'un autre axe de pivotement (98), sur le levier (91) d'une surface de frottement (92) destinée à être appliquée sur une partie de la surface du dispositif de guidage (30) non tournée vers l'autre axe de pivotement (98) et un dispositif élastique (96) qui éloigne la surface de frottement (92) du dispositif de pointage (40), la distance entre le dispositif de pointage (40) et un premier plan (101) perpendiculaire au parcours prédéterminé (38) du dispositif de guidage (30) et un point prévu pour le contact entre la surface de frottement (92) du levier (91) et le dispositif de guidage (30) est plus grande que la distance entre le dispositif de pointage (40) et un deuxième plan (102) perpendiculaire au parcours prédéterminé (38) du dispositif de guidage (30) et qui contient l'autre axe de pivotement (98) du levier (91).
